(19) Europäisches Patentamt
European Patent Office
Office européen des brevets

(11) Veröffentlichungsnummer : **0 069 290**
**B1**

(12) **EUROPÄISCHE PATENTSCHRIFT**

(45) Veröffentlichungstag der Patentschrift : **05.09.84**

(51) Int. Cl.³ : **C 07 D249/08**, C 07 D233/60, A 01 N 43/64, A 01 N 43/50// C07C43/225

(21) Anmeldenummer : **82105597.7**

(22) Anmeldetag : **25.06.82**

# (54) Azolverbindungen, Verfahren zu ihrer Herstellung und diese enthaltende Fungizide.

(30) Priorität : **02.07.81 DE 3126022**

(43) Veröffentlichungstag der Anmeldung : **12.01.83 Patentblatt 83/02**

(45) Bekanntmachung des Hinweises auf die Patenterteilung : **05.09.84 Patentblatt 84/36**

(84) Benannte Vertragsstaaten : **AT BE CH DE FR GB IT LI NL SE**

(56) Entgegenhaltungen : **EP-A- 0 040 350**

(73) Patentinhaber : **BASF Aktiengesellschaft
Carl-Bosch-Strasse 38
D-6700 Ludwigshafen (DE)**

(72) Erfinder : **Sauter, Hubert, Dr.
Neckarpromenade
D-6800 Mannheim (DE)**
Erfinder : **Ammermann, Eberhard, Dr.
Sachsenstrasse 3
D-6700 Ludwigshafen (DE)**
Erfinder : **Rentzea, Costin, Dr.
Neuenheimer Landstrasse 72
D-6900 Heidelberg (DE)**
Erfinder : **Pommer, Ernst-Heinrich, Dr.
Berliner Platz 7
D-6703 Limburgerhof (DE)**

**EP 0 069 290 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue, wertvolle Azolverbindungen, Verfahren zu ihrer Herstellung, diese Verbindungen enthaltende fungizide Mittel und ihre Verwendung als Fungizide.

Es ist bekannt, daß Imidazolderivate, zum Beispiel das 1-[2-(2,4-Dichlorphenyl)-2-(2-propenyloxy)-ethyl]-1H-imidazol (GB-PS 1 318 590) und Triazolderivate, zum Beispiel das 2,2-Dimethyl-4-(1.2.4-triazol-1-yl)-5-phenyl-pentanon-(3) (DE-OS 26 38 470) eine gute fungizide Wirksamkeit zeigen. Die Wirkung ist jedoch bei niedrigen Aufwandmengen und Anwendungskonzentrationen nicht immer befriedigend. Darüber hinaus ist die fungitoxische Wirkung oft mit einer hohen Phytotoxizität verbunden, so daß in den für die Bekämpfung von Pilzen im Pflanzenschutz, beispielsweise bei der Bekämpfung von Rostpilzen notwendigen Konzentrationen auch die Kulturpflanzen geschädigt werden. Aus diesen Gründen sind sie für den Gebrauch als Pflanzenschutzmittel zur Bekämpfung von Pilzen nicht immer und nicht bei allen Pflanzenarten geeignet.

Es wurde nun gefunden, daß Azolverbindungen der Formel I

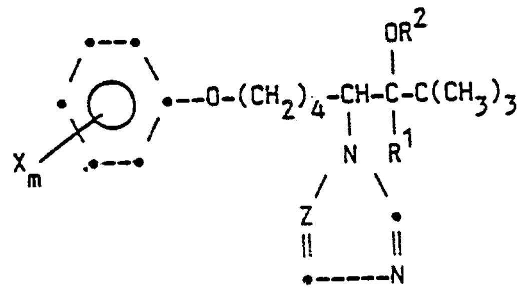

(I)

in welcher

X Wasserstoff, Halogen oder Trifluormethyl und m eine ganze Zahl von 1 bis 5 bedeutet, wobei die einzelnen Gruppen X gleich oder verschieden sind, wenn m größer als 1 ist,

Z N oder CH,

$R^1$ $C_2$-$C_4$-Alkenyl oder $C_2$-$C_4$-Alkinyl und

$R^2$ Wasserstoff, $C_1$-$C_4$-Alkyl, $C_2$-$C_4$-Alkenyl, $C_2$-$C_4$-Alkinyl oder $C_2$-$C_4$-Alkanoyl bedeutet,

sowie deren für Pflanzen verträgliche Säureadditionssalze und Metallkomplexe eine sehr gute fungizide Wirkung bei ausgezeichneter Verträglichkeit durch Pflanzen haben.

In den Verbindungen der Formel I sind das azolylsubstituierte Kohlenstoffatom und das benachbarte, durch Sauerstoff substituierte Kohlenstoffatom chiral ; die Wirkstoffe fallen demgemäß grundsätzlich als Enatiomerengemische an, die in die optisch aktiven Enantiomere getrennt werden können. Im allgemeinen fallen die Wirkstoffe bedingt durch die Anwesenheit zweier chiraler Zentren auch als Diastereomerengemische an, die in üblicher Weise, z. B. durch Kristallisation oder Chromatographie in die einzelnen Komponenten getrennt werden können. Für die Anwendung der Verbindungen als Fungizide ist jedoch eine Trennung der Enantiomeren oder Diastereomeren normalerweise nicht erforderlich. Die Erfindung umfaßt sowohl die Gemische als auch die optisch aktiven einheitlichen Substanzen.

Der Phenoxyrest kann beispielsweise folgendermaßen durch $X_m$ substituiert sein :

Wasserstoff, 2-Fluor-, 4-Fluor-, 2-Chlor-, 3-Chlor-, 4-Chlor-, 4-Brom-, 2,4-Dichlor-, 2,4,6-Trichlor-, 3,5-Dichlor-, 3-Trifluormethyl-, 4-Trifluormethyl-.

$R^1$ bedeutet beispielsweise Vinyl, Propenyl-1, Propenyl-2, Propenyl-3, 2-Methyl-propenyl-3, Ethinyl, Propinyl-1.

$R^2$ bedeutet beispielsweise Wasserstoff, Methyl, Ethyl, n-Propyl, Pro-2-enyl-(1)-, Prop-2-inyl-(1), n-Butyl, 2-Methylprop-2-enyl-(1), Acetyl, Propionyl, Butyryl, Isobutyryl.

Geeignete Säureadditionssalze sind beispielsweise die Chloride, Bromide, Sulfate, Nitrate, Phosphate, Oxalate oder Dodecylbenzolsulfonate. Die Wirksamkeit der Salze geht auf das Kation zurück, so daß die Wahl des Anions beliebig ist.

Geeignete Metallkomplexe sind Verbindungen der Formel

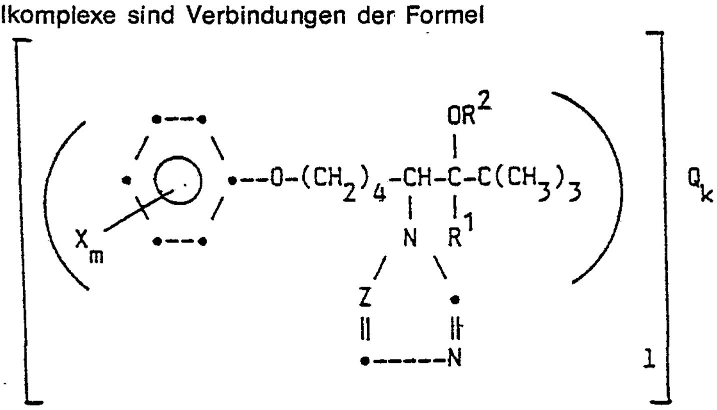

(V)

in der

$X_m$, Z, $R^1$ und $R^2$ die oben angegebenen Bedeutungen haben und Me ein Metall, z. B. Kupfer, Zink, Zinn, Mangan, Eisen, Cobalt oder Nickel bedeutet,

Q für das Anion einer anorganischen Säure steht, z. B. Salzsäure, Schwefelsäure, Phosphorsäure oder Bromwasserstoffsäure und

l und K 1, 2, 3 oder 4 bedeuten.

Die Azolverbindungen der Formel I gemäß Anspruch 1 werden beispielsweise hergestellt, indem man ein Keton der Formel II

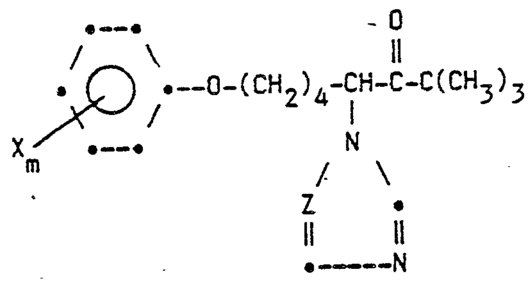

(II)

in der X, m und Z die oben angegebenen Bedeutungen haben mit einer Grignardverbindung der Formel III

$$R^1\text{—MgHal} \qquad \text{(III)}$$

in der $R^1$ für $C_2$-$C_4$-Alkenyl oder $C_2$-$C_4$-Alkinyl steht und in der Hal Chlor, Brom oder lod bedeutet, gegebenenfalls in Gegenwart eines Lösungs- oder Verdünnungsmittels und gegebenenfalls in Gegenwart eines Magnesium- oder Tetraalkylammoniumhalogenids bei Temperaturen zwischen 0 und 100 °C umsetzt und die so entstandenen Alkoholate anschließend zu den tertiären Alkoholen hydrolysiert und die so erhaltenen tertiären Alkohole — wenn $R^2$ verschieden ist von Wasserstoff — mit einem $C_2$-$C_4$-Alkanoylchlorid oder einem $C_2$-$C_4$-Alkanoylanhydrid gegebenenfalls in Gegenwart eines Lösungs- oder Verdünnungsmittels und gegebenenfalls einer anorganischen oder organischen Base, und gegebenenfalls eines Acylierungskatalysators bei Temperaturen zwischen 0 und 100 °C umsetzt oder den tertiären Alkohol oder dessen Alkali- oder dessen quartären Ammoniumsalze mit einem Alkylierungsmittel der Formel IV

$$L\text{—}R^2 \qquad \text{(IV)}$$

in der $R^2$ $C_1$-$C_4$-Alkyl, $C_2$-$C_4$-Alkenyl oder $C_2$-$C_4$-Alkinyl bedeutet und L für eine nucleophile verdrängbare Abgangsgruppe steht, gegebenenfalls in Gegenwart eines Lösungs- oder Verdünnungsmittels und gegebenenfalls anorganischer oder organischer Basen und gegebenenfalls eines Reaktionsbeschleunigers bei Temperaturen zwischen 0 und 100 °C umsetzt und die so erhaltenen Verbindungen der Formel I gegebenenfalls anschließend in ihre für Pflanzen verträgliche Säureadditionssalze oder Metallkomplexe überführt.

Die als Ausgangsprodukte verwendeten Ketone der Formel II lassen sich herstellen, indem man bekannte Ketone der Formel VI (DE-OS 26 38 470) oder deren Alkalienolate mit ω-Aryloxybutylhalogeniden VII, gegebenenfalls in Gegenwart einer Base und gegebenenfalls eines Lösungs- oder Verdünnungsmittels zu den Ketonen der Formel II alkyliert (vgl. Beispiel 1b).

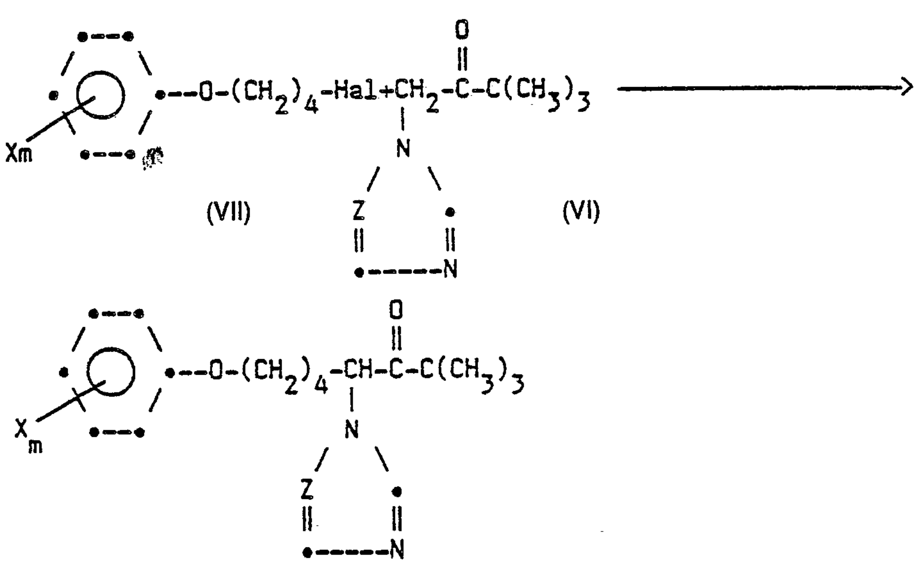

(VII) (VI)

(II)

Hierzu können die Ketone VI zunächst zu den Alkalienolaten metalliert werden, indem man sie vorzugsweise in Gegenwart eines polaren aprotischen Lösungsmittels wie Dimethylformamid, Acetonitril oder Tetrahydrofuran mit 0,8 bis 1,2 Äquivalenten, bevorzugt 1,0 Äquivalenten, eines metallierungsreagens wie Natriumhydrid, Lithiumdiisopropylamid oder n-Butyllithium bei 0 bis 100 °C, vorzugsweise bei 10 bis 50 °C umsetzt. Nach anschließender Zugabe von 0,8 bis 2,0, bevorzugt 1,0 Äquivalenten des jeweiligen ω-Aryl-oxybutylhalogenids der Formel VII erhält man bei Reaktionstemperaturen zwischen 0 und 100 °C, bevorzugt bei 5 bis 30 °C die Ketone der Formel II.

Eine Variante dieses Verfahrens besteht darin, daß man die Ketone VI in Gegenwart von 0,8 bis 1,2, bevorzugt 1,0 Äquivalenten einer Base, z. B. Kalium-tert.-butoxid, Natrium-methoxid oder Kaliumhydroxid mit den ω-Aryloxybutylhalogeniden VII umsetzt, wobei man zweckmäßigerweise in Gegenwart eines Lösungs- oder Verdünnungsmittels bei Temperaturen zwischen 0 und 100 °C, bevorzugt bei 5 bis 50 °C, arbeitet.

Als Lösungsmittel oder Verdünnungsmittel kommen hier wiederum polare aprotische Lösungsmittel in Frage, aber auch Alkohole wie Methanol oder tert.-Butanol.

Die ω-Aryloxybutylhalogenide VII sind bekannte Verbindungen oder können leicht nach bekannten Verfahren hergestellt werden, z. B. durch Monoalkylierung von Phenolen mit aliphatischen Dihalogen alkanen, z. B. mit 1,4-Dibrombutan oder 1,4-Dichlorbutan (s. Houben-Weyl, Methoden der Organischen Chemie, Band 6/3, S. 54-59, Thieme-Verlag, Stuttgart 1965 sowie Beispiel 1a).

Das Verfahren zur Herstellung der tertiären Alkohole der Formel I, in welcher X, m, Y und $R^1$ die oben angegebenen Bedeutungen haben und $R^2$ für Wasserstoff steht, besteht darin, daß man die Ketone der Formel II mit 0,8 bis 2,4 Äquivalenten einer Grignardverbindung der Formel III

$$R^1\text{—MgHal} \qquad \text{(III)}$$

in der $R^1$ $C_2$-$C_4$-Alkenyl oder $C_2$-$C_4$-Alkinyl und Hal Chlor, Brom oder Iod bedeuten, vorzugsweise in Anwesenheit eines Lösungsmittels und gegebenenfalls in Anwesenheit eines ausbeutesteigernden Salzes umsetzt :

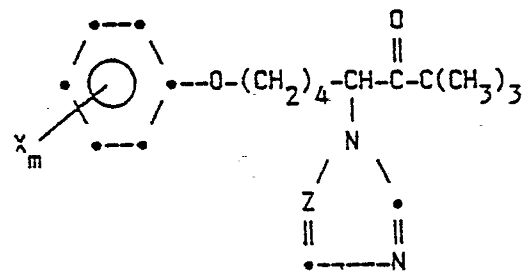

(II)

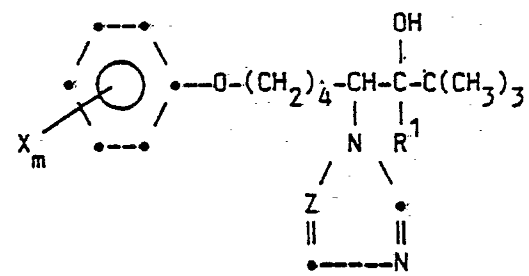

(I, $R^2$ = H)

Als Lösungsmittel kommen bevorzugt Ether in Frage, wie Diethylether, Di-n-propylether, Tetrahydrofuran oder Anisol, ferner tertiäre Amine wie N,N-Diethylanilin sowie Phosphorsäure-tris (dimethylamid) ; gegebenenfalls kann man die Reaktion auch im Gemisch dieser Lösungsmittel mit aliphatischen oder aromatischen Kohlenwasserstoffen wie n-Hexan oder Toluol durchführen. Als ausbeutesteigernde Salze, die die üblichen Nebenreaktionen unterdrücken, kommen insbesondere wasserfreie Magnesiumhalogenide wie wasserfreies Magnesiumbromid oder wasserfreie Tetraalkylammoniumhalogenide wie z. B. Tetra-n-butylammoniumchlorid in Frage. Die Reaktionstemperaturen lassen sich je nach Lösungsmittel zwischen 0 und 100 °C variieren, bevorzugt sind Temperaturen zwischen 0 und 60 °C. Die hierbei primär entstandenen Magnesiumalkoholate werden sodann durch Hydrolyse mit Wasser oder verdünnten wäßrigen Säuren, wie Salzsäure, Schwefelsäure oder bevorzugt Essigsäure oder besonders bevorzugt mit wäßriger Ammoniumchloridlösung in die Alkohole übergeführt, und diese nach Entfernen der wäßrigen Phase, falls gewünscht, in üblicher Weise durch Extraktion, Umkristallisieren oder Chromatographie gereinigt.

Das Verfahren zur Herstellung der Ester der Formel I ($R^2 = C_2$-$C_4$-Alkanoyl) besteht darin, daß man die tertiären Alkohole der Formel I ($R^2 = H$) mit den entsprechenden Säurechloriden oder Säureanhydriden in Gegenwart eines säurebinden den Mittels und gegebenenfalls in Gegenwart eines aprotischen Lösungsmittels oder Verdünnungsmittels sowie bevorzugt in Gegenwart eines Acylierungskatalysators bei Temperaturen zwischen 0 und 100 °C, vorzugsweise zwischen 10 und 50 °C umsetzt. Als säurebindende Mittel können anorganische Basen wie Natriumamid oder besonders bevorzugt Pyridin in mindestens äquivalenten Mengen eingesetzt werden. Als Acylierungskatalysatoren verwendet man zweckmäßigerweise Imidazol oder 4-Dimethylamino-pyridin in Mengenanteilen von 0,01 bis 0,4 Äquivalenten, falls nicht bereits Pyridin anwesend ist. Als Lösungsmittel können Kohlenwasserstoffe, wie Cyclohexan oder Toluol, Ether oder Diethylketon oder auch überschüssige säurebindende Amine wie Triethylamin oder Pyridin eingesetzt werden.

Das Verfahren zur Herstellung der Ether der Formel I ($R^2 = C_1$-$C_4$-Alkyl, $C_2$-$C_4$-Alkenyl oder $C_2$-$C_4$-Alkinyl) besteht darin, daß man die tertiären Alkohole der Formel I ($R^2 = H$) oder ihre Alkali- oder quartären Ammoniumsalze mit dem entsprechenden Alkylierungsmittel der Formel IV

$$L—R^2 \qquad (IV)$$

gegebenenfalls in Gegenwart eines Lösungs- oder Verdünnungsmittels gegebenenfalls in Gegenwart anorganischer oder organischer Basen und gegebenenfalls eines Reaktionsbeschleunigers zwischen 0 und 100 °C umsetzt.

Für die im obigen Verfahren erwähnten nucleophil verdrängbaren Abgangsgruppen L seien beispielsweise genannt: Halogen, vorzugsweise Chlor, Brom oder Iod; Alkylsulfat, vorzugsweise Methylsulfat; gegebenenfalls substituierte Alkylsulfonyloxyreste, vorzugsweise Methansulfonyloxy- oder Trifluormethansulfonyloxyreste oder Arylsulfonyloxyreste, vorzugsweise der Tosylatrest.

Geeignete anorganische oder organische Basen, die gegebenenfalls auch als säurebindende mittel in die Reaktion eingesetzt werden können, sind beispielsweise Alkali- und Erdalkalihydroxide wie Natriumhydroxid, Kaliumhydroxid, Calciumhydroxid, Alkalicarbonate wie Kalium- oder Natriumcarbonat, Alkalihydride wie Natriumhydrid, Alkali- oder Erdalkalialkoholate wie Natriummethylat, Magnesiummethylat oder Natriumisopropylat oder tertiäre Amine wie Trimethylamin, Triethylamin, N,N-Dimethylanilin, N,N-Dimethylcyclohexyl-amin, N-Methylpiperidin oder Pyridin. Es können aber auch andere übliche Basen verwendet werden.

Mit geeigneten Basen wie z. B. Alkalihydrid wie Natriumhydrid oder Lithiumalkylen wie Butyllithium oder mit Alkali- oder Erdalkalialkoholaten wie Natriummethylat können die tertiären Alkohole der Formel I ($R^2 = H$) auch in einer vorgeschalteten Umsetzung zunächst in ihre Alkoholate überführt werden und dann als solche zur Reaktion gebracht werden.

Zu den bevorzugten Lösungs- bzw. Verdünnungsmitteln gehören Halogenkohlenwasserstoffe wie beispielsweise Methylenchlorid, Chloroform, 1,2-Dichlorethan, Chlorbenzol; aliphatische oder aromatische Kohlenwasserstoffe wie Cylohexan, Petrolether, Benzol, Toluol oder Xylole, Ester wie Essigsäureethylester, Amide wie Dimethylformamid, Nitrile wie Acetonitril, Sulfoxide wie Dimethylsulfoxid, Ketone wie Aceton oder Methylethylketon, Ether wie Diethylether, Tetrahydrofuran oder Dioxan oder entsprechende Gemische.

Als Reaktionsbeschleuniger kommen vorzugsweise Metallhalogenide wie Kaliumiodid, Kronenether, quartäre Ammoniumverbindungen wie Tetrabutylammoniumiodid oder Säuren oder Kombinationen dieser Reaktionsbeschleuniger in Frage.

Die Umsetzungen werden im allgemeinen bei Temperaturen zwischen 0 und 100 °C in einem Zeitraum von 1 bis 60 Stunden durchgeführt, drucklos oder unter Druck, kontinuierlich oder diskontinuierlich.

Zur Isolierung der neuen Verbindungen wird nach üblichen Methoden vorgegangen. Im allgemeinen bedürfen die anfallenden Produkte keiner weiteren Reinigung, sie können aber nach bekannten Methoden wie Umkristallisation, Extraktion, Destillation oder Chromatographie weiter gereinigt werden.

Falls gewünscht, können die neuen Verbindungen der Formel I auch in Salze mit anorganischen oder organisschen Säuren übergeführt werden, wie beispielsweise in Salze der Salzsäure, Bromwasserstoffsäure, Salpetersäure, Oxalsäure, Essigsäure, Schwefelsäure, Phosphorsäure oder Dodecylbenzosulfonsäure. Die Wirksamkeit der Salze geht auf das Kation zurück, so daß die Wahl des Anions beliebig ist.

Ferner lassen sich die Verbindungen der Formel I nach bekannten Methoden in Metallkomplexe überführen. Das kann durch Umsetzung dieser Verbindungen mit geeigneten Metallsalzen wie beispielsweise Kupfer (II)-chlorid, Zink (II)-chlorid, Eisen (III)-chlorid, Kupfer (II)-nitrat, Mangan (II)-chlorid oder Nickel (II)-bromid erfolgen.

Die Herstellung der neuen Verbindungen der Formel I wird durch folgende Beispiele erläutert;

## Beispiel 1

a) Herstellung des Zwischenproduktes 1-Brom-4-phenoxybutan

Eine Mischung von 329 g Phenol, 484 g trockenem Kaliumcarbonat, 756 g 1,4-Dibrombutan und

1 000 ml Cyclopentanon wird unter Rühren 24 Stunden unter Rückfluß zum Sieden erhitzt. Nach Abfiltrieren der festen Bestandteile wird das Filtrat i. Vak. eingeengt, der ölige Rückstand in 1 500 ml Dichlormethan aufgenommen und zehnmal mit je 200 ml 15 proz. (Gew.%) wäßriger Natriumhydroxidlösung extrahiert. Nach zweimaliger Extraktion der organischen Phase mit je 300 ml Wasser trocknet man sie über Magnesiumsulfat, filtriert und engt i. Vak. ein. Aus dem Rückstand destilliert man bei 92 bis 98 °C/0,4 mbar 395 g farbloses 1-Brom-3-phenoxybutan, das in der Vorlage erstarrt (Fp. 33 bis 36 °C).

b) Herstellung des Zwischenproduktes 2,2-Dimethyl-4-(1,2,4-triazol-1-yl)-8-phenoxy-octanon-(3)

Zur gerührten Suspension von 2,3 g Natriumhydrid in 20 ml Dimethylformamid (DMF) tropft man unter einer trockenen Stickstoffatmosphäre eine Lösung von 14,3 g 2,2-Dimethyl-4-(1,2,4-triazol-1-yl)-butanon-(3) (siehe DE-OS 26 38 470) in 20 ml DMF, wobei die Reaktionstemperatur durch Kühlung zwischen 20° und 30 °C gehalten wird und rührt anschließend noch 20 Stunden bei Raumtemperatur. Dann tropft man unter weiterem Rühren und Eiskühlung eine Lösung von 19,5 g 1-Brom-4-phenoxybutan in 20 ml DMF bei 5° bis 10 °C zu. Nach Beendigung der Zugabe rührt man noch 10 Stunden bei 5° bis 10 °C weiter, fügt sodann 200 ml Wasser hinzu und extrahiert das Gemisch danach zweimal mit je 100 ml Dichlormethan. Aus den vereinigten organischen Phasen gewinnt man nach Ausschütteln mit Wasser, Trocknen über Magnesiumsulfat und Entfernen des Lösungsmittels i. Vak. ein Öl, aus dem beim Anreiben mit 20 ml Diisopropylether 17 g farbloser Kristalle ausfallen, Fp 59 bis 62 °C.

c) Herstellung von 2,2-Dimethyl-3-vinyl-4-(1,2,4-triazol-1-yl)-8-phenoxy-octanol-(3) (Verbindung Nr. 1).

Zur Lösung von 8,7 g Vinylmagnesiumchlorid in 70 ml Tetrahydrofuran tropfte man unter Rühren eine Lösung von 12,6 g 2,2-Dimethyl-4-(1,2,4-triazol-1-yl)-8-phenoxy-octanon-(3). Nach Abklingen der schwach exothermen Reaktion erhitzte man die Mischung noch 5 Stunden auf Rückflußtemperatur. Nach dem Abkühlen wurde unter Eiskühlung eine Mischung von 5 ml Wasser und 5 ml Tetrahydrofuran zugetropft, das Reaktionsgemisch noch 2 Stunden bei Raumtemperatur gerührt, anschließend von den ausgefallenen Magnesiumsalzen abfiltriert und das Filtrat i. Vak. eingeengt. Der Rückstand des Filtrats wurde mit 100 ml Dichlormethan aufgenommen, mit 100 ml Wasser und anschließend mit 100 ml gesättigter Ammoniumchloridlösung gewaschen und die organische Phase nach dem Trocknen über Magnesiumsulfat i. Vak. eingeengt. Aus dem zurückbleibenden Öl kristallisierten beim Anreiben mit Diisopropylether 8,0 g blaß gelbliche Kristalle vom Fp. 66-69 °C (Diastereomerengemisch 2 : 1).

Weitere Beispiele für Verbindungen der Formel I, die entsprechend hergestellt wurden, sind in der folgenden Tabelle enthalten.

Tabelle 1

| Verb. Nr. | $X_m$ | Z | $R^1$ | $R^2$ | Fp (°C) |
|---|---|---|---|---|---|
| 2 | H | N | $-CH_2-CH=CH_2$ | H | IR (Film): 3250, 2960, 1595, 1490, 1240, 1075, 915, 755, 695, 670 $cm^{-1}$ |
| 3 | H | N | -ethinyl | H | 129 - 132 |
| 4 | H | N | -propinyl-1 | H | 113 - 115 |
| 5 | H | CH | $-CH=CH_2$ | H | 108 - 111 |
| 6 | H | CH | $-CH_2-CH=CH_2$ | H | IR (Film): 3350, 2960, 1595, 1495, 1240, 1135, 1010, 915, 750, 685 $cm^{-1}$ |
| 7 | H | CH | -ethinyl | H | 156 - 158 |
| 8 | H | CH | -propinyl-1 | H | 107 - 110 |
| 9 | 4-F | N | $-CH=CH_2$ | H | 73 - 75 |
| 10 | 4-F | N | $-CH_2-CH=CH_2$ | H | IR (Film): 3350, 2960, 1500, 1200, 1135, 1010, 825, 760, 685, 500 $cm^{-1}$ |
| 11 | 4-F | N | -ethinyl | H | 110 - 112 |
| 12 | 4-F | N | -propinyl-1 | H | 123 - 126 |

Tabelle I (Fortsetzung)

| Verb. Nr. | $X_m$ | Z | $R^1$ | $R^2$ | Fp (°C) |
|---|---|---|---|---|---|
| 13 | 4-Cl | N | $-CH=CH_2$ | H | IR (Film): 3400, 2960, 1595, 1490, 1280, 1250, 1140, 1010, 825, 665 $cm^{-1}$ |
| 14 | $3-CF_3$ | N | $-CH=CH_2$ | H | IR (Film): 3400, 2960, 1590, 14,50, 1325, 1240, 1170, 1130, 790, 700 $cm^{-1}$ |
| 15 | 3-Cl | N | $-CH=CH_2$ | H | IR (Film): 3400, 2960, 1595, 1280, 1250, 1230, 1140, 770, 680 $cm^{-1}$ |
| 16 | $2,4-Cl_2$ | N | $-CH=CH_2$ | H | 86 - 88 |
| 17 | $2,4-Cl_2$ | N | -ethinyl | H | 123 - 125 |

Die neuen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie in Gemüsen wie Gurken, Bohnen oder Kürbisgewächsen.

Die neuen Verbindungen sind insbesondere geeignet zur Bekämpfung folgender Pflanzenkrankheiten :

Erysiphe graminis (echter Mehltau) in Getreide,
Erysiphe cichoriacearum (echter Mehltau) an Kürbisgewächsen,
Podosphaera leucotricha an Äpfeln,
Uncinula necator an Reben,
Erysiphe polygoni an Bohnen,
Sphaerotheca pannosa an Rosen,
Puccinia-Arten an Getreide,
Rhizoctonia solani an Baumwolle sowie
Helminthosporiumarten an Getreide,
Ustilago-Arten an Getreide und Zuckerrohr,
Botrytis cinerea an Reben,
Monilia fructigena an Äpfeln,
Rhynchosporium secale an Getreide und ganz besonders
Venturia inaequalis (Apfelschorf).

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Ketone (z. B. Cyclohexanon), Amine (z. B. Ethanolamin, Dimethylformamid) und Wasser ; Trägerstoffe wie natürliche Gesteinsmehle (z. B. Kaoline, Tonerden, Talkum, Kreide) und synthetische Gesteinsmehle (z. B. hochdisperse Kieselsäure, Silikate) ; Emulgiermittel wie nichtionogene

und anionische Emulgatoren (z. B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch in Materialschutz u. a. zur Bekämpfung holzzerstörender Pilze wie Coniophora puteanea und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind :

I. Man vermischt 90 Gewichtsteile der Verbindung Nr. 1 mit 10 Gewichtsteilen N-Methyl-alphapyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanol-amid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid and 1 Mol Ricinusöl besteht. Durch Eingießen und feines verteilen der Lösung in Wasser erhält man eine wäßrige Dispersion.

IV. 20 Gewichtsteile der Verbindung Nr. 6 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 8 werden den mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 9 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 5 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 6 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise :

Schwefel,
Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat und
Zinkethylenbisdithiocarbamat,
Tetramethylthiuramdisulfide,
Ammoniak-Komplex von Zink-(N,N-ethylen-bis-dithiocarbamat) und N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und N,N'-Polypropylen-bis-(thiocarbamoyl)-disulfid ;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;

heterocyclische Substanzen, wie
N-(1,1,2,2-Tetrachlorethylthio)-tetrahydrophthalimid,
N-Trichlormethylthio-tetrahydrophthalimid,
N-Trichlormethylthio-phthalimid,
2-Heptadecyl-2-imidazolin-acetat,
2,4-Dichlor-6-(o-chloranilino)-s-triazin,
O,O-Diethyl-phthalimidophosphonothioat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin, 2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und weiter Substanzen wie
Dodecylguanidinacetat,
3-(3-(3,5-dimethyl-2-oxycyclohexyl)-2-hydroxyethyl)-glutaramid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Cyan-N-(ethylaminocarbonyl)-2-(methoxyimino)-acetamid,
2-Methyl-benzoesäure-anilid,
2-Iod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze.

DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,

5-Nitro-isophthalsäure-di-isopropylester,
1-(1',2',4'-Triazolyl-1')-1-(4'-chlorphenoxy)-3,3-dimethylbutan-2-on,
1-(1',2',4'-Triazolyl-1')-1-(4'-chlorphenoxy)-3,3-dimethylbutan-2-ol,

N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff.

Die folgenden Versuche 1-5 zeigen die fungizide Wirkung der neuen Substanzen. Als Vergleichssubstanzen dienten (1-(2'-(2",4"-Dichlorphenyl)-2'-(2"-propenyloxy)-ethyl)-1H-imidazol) (A) sowie das 2,2-Dimethyl-4-(1,2,4-triazol-1-yl)-5-phenyl-pentanon-(3) (DE-OS 26 38 470) (B).

Versuch 1

Wirksamkeit gegen Weizenmehltau

Blätter von in Töpfen gewachsenen Weizenkeimlingen der Sorte « Jubilar » werden mit wäßrigen Emulsionen aus 80 % (Gew.%) Wirkstoff und 20 % Emulgiermittel (Na-Lignin-Sulfonat) besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22 °C und 75 bis 80 % relativer Luftfeuchtigkeit aufgestellt. Nach 7 Tagen wird das Ausmaß der Mehltauentwicklung ermittelt.

Das Ergebnis des Versuches zeigt, daß beispielsweise die Wirkstoffe 1, 2, 5, 6, 8, 9,10, 12, 13, 14, 15 und 16 beispielsweise bei Anwendung als 0,025 %ige und 0,006 %ige wäßrige Spritzbrühe eine bessere fungizide Wirkung haben (beispielsweise 100 %ige Wirkung) als das Vergleichsmittel A (beispielsweise 40 %ige Wirkung).

Versuch 2

Wirksamkeit gegen Gurkenmehltau

Blätter von in Töpfen gewachsenen Gurkenkeimlingen der Sorte « Chinesische Schlange » werden im Zweiblattstadium mit einer Sporensuspension des Gurkenmehltaus (Erysiphe cichoracearum) besprüht. Nach etwa 20 Stunden werden die Versuchspflanzen mit wäßrigen Emulsionen aus 80 % Wirkstoff und 20 % Emulgiermittel (Na-Lignin-Sulfonat) bis zur Tropfnässe besprüht. Nach dem Antrocknen des Spritzbelages werden sie anschließend im Gewächshaus bei Temperaturen zwischen 20 und 22 °C und 70 bis 80 % relativer Luftfeuchtigkeit aufgestellt. Zur Beurteilung der Wirksamkeit der neuen Wirkstoffe wird das Ausmaß der Pilzentwicklung nach 21 Tagen beurteilt.

Das Ergebnis des Versuches zeigt, daß beispielsweise die Wirkstoffe 1, 2, 3, 5, 6, 7, 8, 9, 10, 11, 12, 13, 14, 15, 16 und 17 bei Anwendung als 0,025 %ige Spritzbrühe eine bessere fungizide Wirkung (beispielsweise 100 %ige Wirkung) haben als das Vergleichsmittel B (beispielsweise 40 %ige Wirkung).

Versuch 3

Wirksamkeit gegen Weizenbraunrost

Blätter von in Töpfen gewachsenen Weizensämlingen der Sorte « Jubilar » werden mit Sporen des Braunrostes (Puccinia recondita) bestäubt. Danach werden die Töpfe für 24 Stunden bei 20 bis 22 °C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel (Na-Lignin-Sulfonat) in der Trockensubstanz enthalten, tropfnaß gespritzt. Nach dem Antrocknen des Spritzbelages werden die Versuchspflanzen im Gewächshaus bei Temperaturen zwischen 20 und 22 °C und 65 bis 70 % relativer Luftfeuchte aufgestellt. Nach 8 Tagen wird das Ausmaß der Rostpilzentwicklung auf den Blättern ermittelt.

Das Ergebnis des Versuches zeigt, daß beispielsweise die Wirkstoffe 1, 2, 5, 6, 8, 9, 10, 13, 14, 15 und 16 beispielsweise bei Anwendung als 0,025 %ige und 0,006 %ige wäßrige Spritzbrühe eine bessere fungizide Wirkung (beispielsweise 100 %ige Wirkung) haben als das Vergleichsmittel A (beispielsweise 20 %ige Wirkung).

Versuch 4

Wirksamkeit gegen Botrytis cinerea an Paprika

Paprikasämlinge der Sorte « Neusiedler Ideal Elite » werden, nachdem sich 4 bis 5 Blätter gut entwickelt haben, mit 0,05 % (Gew.%) wäßrigen Suspensionen, die 80 % Wirkstoff und 20 % Emulgiermittel (Na-Lignin-Sulfonat) in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24 °C in eine Kammer mit hoher Luftfeuchtigkeit gestellt, um optimale Bedingungen für die Pilzentwicklung zu erhalten. Nach 5 Tagen hat sich die Krankheit auf den

unbehandelten Kontrollpflanzen so stark entwickelt, daß die entstandenen Blattnekrosen den überwiegenden Teil der Blätter bedecken.

Das Ergebnis des Versuches zeigt, daß die Wirkstoffe 1, 2, 5, 6, 8 und 10 bei Anwendung als 0,05 %ige wäßrige Spritzbrühe eine gute fungizide Wirkung haben.

Das Ergebnis des Versuches zeigt, daß beispielsweise die Wirkstoffe 1, 2, 5, 6, 8 und 10 bei Anwendung als 0,05 %ige wäßrige Spritzbrühe eine gute fungizide Wirkung (beispielsweise 100 %ige Wirkung) haben.

Versuch 5

Wirksamkeit gegen Apfelschorf

Junge Blätter von in Töpfen gewachsenen Apfelsämlingen der Sorte « Golden Delicious » werden mit wäßriger Spritzbrühe, die 80 % Wirkstoff und 20 % Emulgiermittel (Na-Lignin-Sulfonat) in der Trockensubstanz enthält, tropfnaß gespritzt. Nach dem Antrocknen des Spritzbelages werden die Versuchspflanzen mit einer Sporensuspension des Apfelschorfs (Venturia inaequalis) besprüht. Anschließend werden die inoculierten Pflanzen in einer Klimakammer bei 20 bis 22 °C und 95 % relativer Luftfeuchtigkeit für 10 Tage aufgestellt. Dann wird das Ausmaß der Pilzentwicklung auf den Blättern ermittelt.

Das Ergebnis des Versuches zeigt, daß beispielsweise die Wirkstoffe 1 und 2 bei Anwendung als 0,075 %ige wäßrige Spritzbrühe eine gute fungizide Wirkung (beispielsweise 100 %ige Wirkung) haben.

**Ansprüche** (für die Vertragsstaaten : BE, CH, DE, FR, GB, IT, LI, NL, SE)

1. Azolverbindung der Formel I

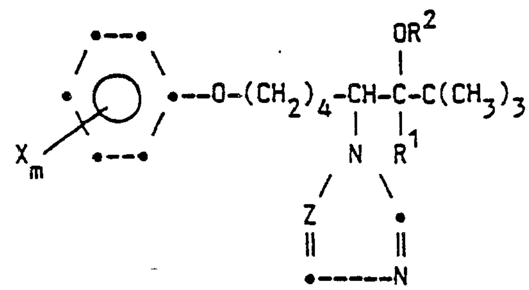

(I)

in welcher

X Wasserstoff, Halogen oder Trifluormethyl und m eine ganze Zahl von 1 bis 5 bedeutet, wobei die einzelnen Gruppen X gleich oder verschieden sind, wenn m größer als 1 ist,

Z N oder CH,

$R^1$ $C_2$-$C_4$-Alkenyl oder $C_2$-$C_4$-Alkinyl und

$R^2$ Wasserstoff, $C_1$-$C_4$-Alkyl, $C_2$-$C_4$-Alkenyl, $C_2$-$C_4$-Alkinyl oder $C_2$-$C_4$-Alkanoyl bedeutet,

sowie ihre für Pflanzen verträgliche Säureadditionssalze und Metallkomplexe.

2. Fungizid enthaltend eine Azolverbindung gemäß Anspruch 1.

3. Fungizid enthaltend einen festen oder flüssigen Trägerstoff und eine Azolverbindung gemäß Anspruch 1.

4. Verfahren zur Herstellung eines Fungizids, dadurch gekennzeichnet, daß man einen festen oder flüssigen Trägerstoff vermischt mit einer Azolverbindung gemäß Anspruch 1.

5. Verfahren zur Bekämpfung von Pilzen, dadurch gekennzeichnet, daß man die Pilze oder die vor Pilzbefall zu schützenden Gegenstände behandelt mit einer Azolverbindung gemäß Anspruch 1.

6. Verfahren zur Herstellung einer Azolverbindung gemäß Anspruch 1, dadurch gekennzeichnet, daß man ein Keton der Formel II

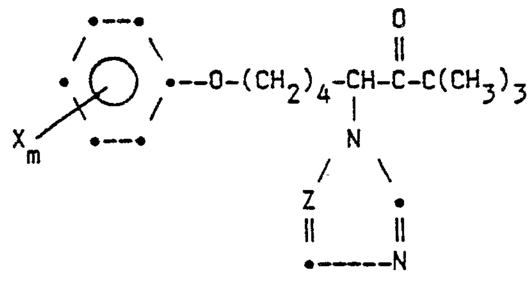

(II)

in der X, m und Z die im Anspruch 1 angegebenen Bedeutungen haben mit einer Grignardverbindung der Formel III

$$R^1—MgHal \quad (III)$$

in der $R^1$ $C_2$-$C_4$-Alkenyl oder $C_3$-$C_4$-Alkinyl bedeutet und in der Hal Chlor, Brom oder Iod bedeutet, gegebenenfalls in Gegenwart eines Lösungs oder Verdünnungsmittels und gegebenenfalls in Gegenwart eines Magnesium- oder Tetraalkylammoniumhalogenids bei Temperaturen zwischen 0 und 100 °C umsetzt und das so erhaltene Alkoholat anschließend zu dem tertiären Alkohol hydrolysiert und den so erhaltenen tertiären Alkohol — wenn $R^2$ verschieden ist von Wasserstoff — mit einem $C_2$-$C_4$-Alkanoylchlorid oder einem $C_2$-$C_4$-Alkanoylanhydrid gegebenenfalls in Gegenwart eines Lösungs- oder Verdünnungsmittels und gegebenenfalls einer anorganischen oder organischen Base, und gegebenenfalls eines Acylierungskatalysators bei Temperaturen zwischen 0 und 100 °C umsetzt oder den tertiären Alkohol oder dessen Alkali- oder dessen quartären Ammoniumsalz mit einem Alkylierungsmittel der Formel IV

$$L—R^2 \quad (IV)$$

in der $R^2$ $C_1$-$C_4$-Alkyl, $C_2$-$C_4$-Alkenyl oder $C_2$-$C_4$-Alkinyl bedeutet und L für eine nucleophil verdrängbare Abgangsgruppe steht, gegebenenfalls in Gegenwart eines Lösungs- oder Verdünnungsmittels und gegebenenfalls einer anorganischen oder organischen Base und gegebenenfalls eines Reaktionsbeschleunigers bei Temperaturen zwischen 0 und 100 °C umsetzt und die so erhaltenen Verbindungen der Formel I gegebenenfalls anschließend in ihre für Pflanzen verträgliche Säureadditionssalze oder Metallkomplexe überführt.

**Ansprüche** (für den Vertragsstaat AT)

1. Fungizid enthaltend eine Azolverbindung der Formel I

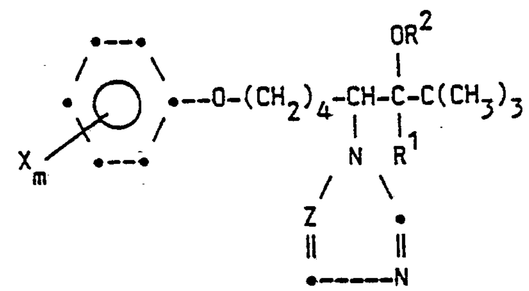

(I)

in welcher

X Wasserstoff, Halogen oder Trifluormethyl und m eine ganze Zahl von 1 bis 5 bedeutet, wobei die einzelnen Gruppen X gleich oder Verschieden sind, wenn m größer als 1 ist,

Z N oder CH,

$R^1$ $C_2$-$C_4$-Alkenyl oder $C_2$-$C_4$-Alkinyl und

$R^2$ Wasserstoff, $C_1$-$C_4$-Alkyl, $C_2$-$C_4$-Alkenyl, $C_2$-$C_4$-Alkinyl oder $C_2$-$C_4$-Alkanoyl bedeutet,

sowie ihre für Pflanzen verträgliche Säureadditionssalze und Metallkomplexe.

2. Fungizid enthaltend einen festen oder flüssigen Trägerstoff und eine Azolverbindung gemäß Anspruch 1.

3. Verfahren zur Herstellung eines Fungizids, dadurch gekennzeichnet, daß man einen festen oder flüssigen Trägerstoff vermischt mit einer Azolverbindung gemäß Anspruch 1.

4. Verfahren zur Bekämpfung von Pilzen, dadurch gekennzeichnet, daß man die Pilze oder die vor Pilzbefall zu schützenden Gegenstände behandelt mit einer Azolverbindung gemäß Anspruch 1.

5. Verfahren zur Herstellung einer Azolverbindung gemäß Anspruch 1, dadurch gekennzeichnet, daß man ein Keton der Formel II

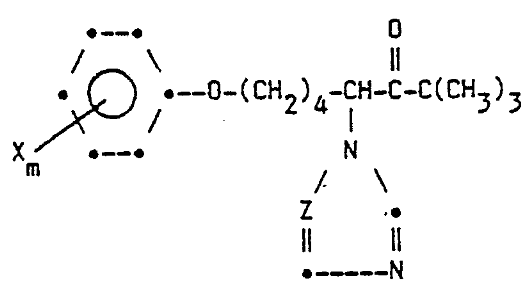

(II)

in der X, m und Z die im Anspruch 1 angegebenen Bedeutungen haben mit einer Grignardverbingung der Formel III

$$R^1—MgHal \qquad (III)$$

in der $R^1$ $C_2$-$C_4$-Alkenyl oder $C_3$-$C_4$-Alkinyl bedeutet und in der Hal Chlor, Brom oder Iod bedeutet, gegebenenfalls in Gegenwart eines Lösungs- oder Verdünnungsmittels und gegebenenfalls in Gegenwart eines Magnesium- oder Tetraalkylammoniumhalogenids bei Temperaturen zwischen 0 und 100 °C umsetzt und das so erhaltene Alkoholat anschließend zu dem tertiären Alkohol hydrolysiert und den so erhaltenen tertiären Alkohol — wenn $R^2$ verschieden ist von Wasserstoff — mit einem $C_2$-$C_4$-Alkanoylchlorid oder einem $C_2$-$C_4$-Alkanoylanhydrid gegebenenfalls in Gegenwart eines Lösungs- oder Verdünnungsmittels und gegebenenfalls einer anorganischen oder organischen Base, und gegebenenfalls eines Acylierungskatalysators bei Temperaturen zwischen 0 und 100 °C umsetzt oder den tertiären Alkohol oder dessen Alkali- oder dessen quartären Ammoniumsalz mit einem Alkylierungsmittel der Formel IV

$$L—R^2 \qquad (IV)$$

in der $R^2$ $C_1$-$C_4$-Alkyl, $C_2$-$C_4$-Alkenyl oder $C_2$-$C_4$-Alkinyl bedeutet und L für eine nucleophil verdrängbare Abgangsgruppe steht, gegebenenfalls in Gegenwart eines Lösungs- oder Verdünnungsmittels und gegebenenfalls einer anorganischen oder organischen Base und gegebenenfalls eines Reaktionsbeschleunigers bei Temperaturen zwischen 0 und 100 °C umsetzt und die so erhaltenen Verbindungen der Formel I gegebenenfalls anschließend in ihre für Pflanzen verträgliche Säureadditionssalze oder Metallkomplexe überführt.

**Claims** (for the Contracting States : BE, CH, DE, FR, GB, IT, LI, NL, SE)

1. An azole compound of the formula I

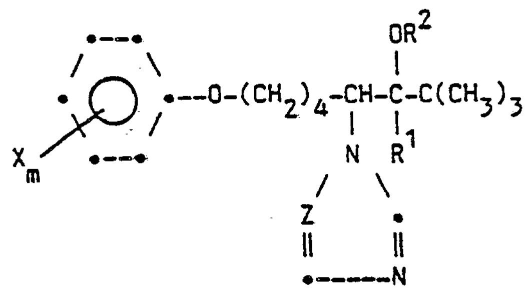

(I)

where

X is hydrogen, halogen or trifluoromethyl, m is an integer from 1 to 5, and, if m is greater than 1, the X's can be identical or different,

Z is N or CH,

$R^1$ is $C_2$-$C_4$-alkenyl or $C_2$-$C_4$-alkynyl, and

$R^2$ is hydrogen, $C_1$-$C_4$-alkyl, $C_2$-$C_4$-alkenyl, $C_2$-$C_4$-alkynyl or $C_2$-$C_4$-alkanoyl,

or a crop-tolerated addition salt thereof with an acid, or a metal complex thereof.

2. A fungicide containing an azole compound as claimed in claim 1.

3. A fungicide containing a solid or liquid carrier and an azole compound as claimed in claim 1.

4. A process for the production of a fungicide, wherein a solid or liquid carrier is mixed with an azole compound as claimed in claim 1.

5. A process for combating fungi, wherein the fungi or the objects to be protected against fungus attack are treated with an azole compound as claimed in claim 1.

6. A process for the preparation of an azole compound as claimed in claim 1, wherein a ketone of the formula II

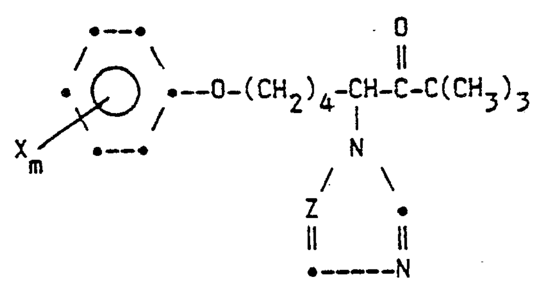

(II)

where X, m and Z have the meanings given in claim 1, is reacted with a Grignard compound of the formula III

$$R^1—MgHal \qquad (III)$$

where $R^1$ is $C_2$-$C_4$-alkenyl or $C_2$-$C_4$-alkynyl and Hal is chlorine, bromine or iodine, in the presence or absence of a solvent or diluent and in the presence or absence of a magnesium halide or tetraalkylammonium halide, at from 0 to 100 °C, and the resulting alcoholate is then hydrolyzed to the tertiary alcohol, and the tertiary alcohol thus obtained — where $R^2$ is not hydrogen — is reacted with a $C_2$-$C_4$-alkanoyl chloride or with a $C_2$-$C_4$-alkanoyl anhydride, in the presence or absence of a solvent or diluent, of an inorganic or organic base, and of an acylation catalyst, at from 0 to 100 °C, or the tertiary alcohol or its alkali metal salt or quaternary ammonium salt is reacted with an alkylating agent of the formula IV

$$L—R^2 \qquad (IV)$$

where $R^2$ is $C_1$-$C_4$-alkyl, $C_2$-$C_4$-alkenyl or $C_2$-$C_4$-alkynyl and L is a nucleophilically displaceable leaving group, in the presence or absence of a solvent or diluent, of an inorganic or organic base, and of a reaction accelerator, at from 0 to 100 °C, and the compound of the formula I thus obtained is, if desired, subsequently converted into a crop-tolerated acid addition salt or a metal complex.

**Claims** (for the Contracting State AT)

1. A fungicide containing an azole compound of the formula I

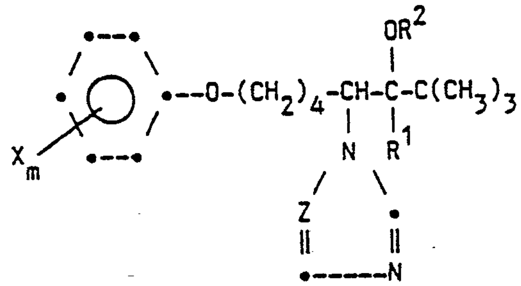

(I)

where

X is hydrogen, halogen or trifluoromethyl, m is an integer from 1 to 5, and, if m is greater than 1, the X's can be identical or different,

Z is N or CH,

$R^1$ is $C_2$-$C_4$-alkenyl or $C_2$-$C_4$-alkynyl, and

$R^2$ is hydrogen, $C_1$-$C_4$-alkyl, $C_2$-$C_4$-alkenyl, $C_2$-$C_4$-alkynyl or $C_2$-$C_4$-alkanoyl,

or a crop-tolerated addition salt thereof with an acid, or a metal complex thereof.

2. A fungicide containing a solid or liquid carrier and an azole compound as defined in claim 1.

3. A process for the production of a fungicide, wherein a solid or liquid carrier is mixed with an azole compound as defined in claim 1.

4. A process for combating fungi, wherein the fungi or the objects to be protected against fungus attack are treated with an azole compound as defined in claim 1.

5. A process for the preparation of an azole compound as defined in claim 1, wherein a ketone of the formula II

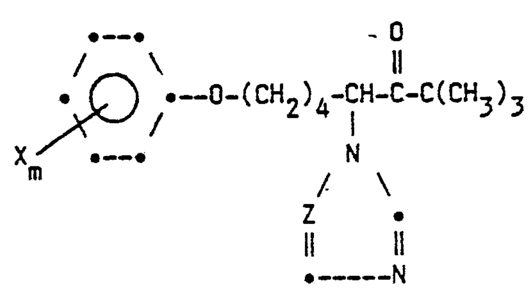

(II)

where X, m and Z have the meanings given in claim 1, is reacted with a Grignard compound of the formula III

$$R^1—MgHal \qquad (III)$$

where $R^1$ is $C_2$-$C_4$-alkenyl or $C_2$-$C_4$-alkynyl and Hal is chlorine, bromine or iodine, in the presence or absence of a solvent or diluent and in the presence or absence of a magnesium halide or tetraalkylammonium halide, at from 0 to 100 °C, and the resulting alcoholate is then hydrolyzed to the tertiary alcohol, and the tertiary alcohol thus obtained — where $R^2$ is not hydrogen — is reacted with a $C_2$-$C_4$-alkanoyl chloride or with a $C_2$-$C_4$-alkanoyl anhydride, in the presence or absence of a solvent or diluent, of an inorganic or organic base, and of an acylation catalyst, at from 0 to 100 °C, or the tertiary alcohol or its alkali metal salt or quaternary ammonium salt is reacted with an alkylating agent of the formula IV

$$L—R^2 \qquad (IV)$$

where $R^2$ is $C_1$-$C_4$-alkyl, $C_2$-$C_4$-alkenyl or $C_2$-$C_4$-alkynyl and L is a nucleophilically displaceable leaving group, in the presence or absence of a solvent or diluent, of an inorganic or organic base, and of a reaction accelerator, at from 0 to 100 °C, and the compound of the formula I thus obtained is, if desired, subsequently converted into a crop-tolerated acid addition salt or a metal complex.

**Revendications** (pour les Etats contractants : BE, CH, DE, FR, GB, IT, LI, NL, SE)

1. Dérivé d'azole de formule I

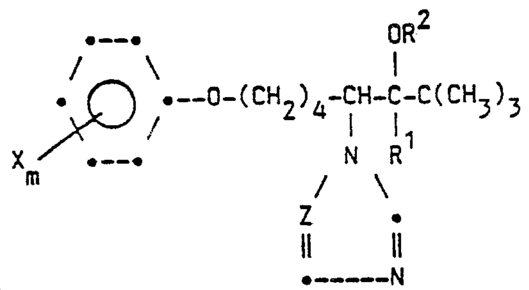

(I)

dans laquelle

X représente hydrogène, halogène ou trifluorométhyle et m un nombre entier de 1 à 5, les différents groupes X étant identiques ou différents quand m est supérieur à 1,

Z représente N ou CH,

$R^1$ représente un alcenyle en $C_2$-$C_4$ ou un alcinyle en $C_2$-$C_4$ et

$R^2$ représente hydrogène, alkyle en $C_1$-$C_4$, alcényle en $C_2$-$C_4$, alcinyle en $C_2$-$C_4$ ou alcanoyle en $C_2$-$C_4$

ainsi que ses sels d'addition d'acide et complexes métalliques, compatibles avec des plantes.

2. Fongicide contenant un dérivé d'azole selon la revendication 1.

3. Fongicide contenant un support solide ou liquide et un dérivé d'azole selon la revendication 1.

4. Procédé de préparation d'un fongicide, caractérisé par le fait qu'on mélange un support solide ou liquide avec un dérivé d'azole selon la revendication 1.

5. Procédé de lutte contre les champignons, caractérisé par le fait qu'on traite les champignons, ou les objets à protéger contre les champignons, avec un dérivé d'azole selon la revendication 1.

6. Procédé de préparation d'un dérivé d'azole selon la revendication 1 caractérisé par le fait qu'on fait réagir, éventuellement en présence d'un solvant ou diluant et éventuellement en présence d'un halogénure de magnésium ou de tétraalkylammonium, à des températures comprises entre 0 et 100 °C, une cétone de formule II

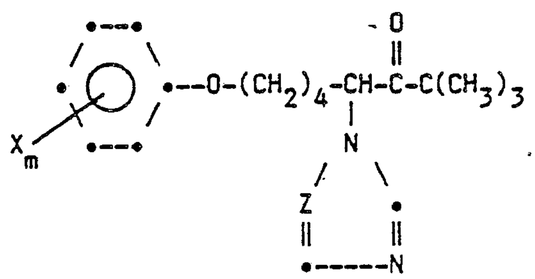

(II)

dans laquelle X, m et Z ont les significations données dans la revendication 1, avec un composé de Grignard de formule III

$$R^1—MgHal \qquad (III)$$

dans laquelle $R^1$ représente alcényle en $C_2$-$C_4$ ou alcinyle en $C_3$-$C_4$ et où Hal représente chlore, brome ou iode, et l'alcoolat ainsi obtenu est ensuite hydrolysé en alcool tertiaire et l'alcool tertiaire ainsi obtenu — quand $R^2$ est différent d'hydrogène — est mis à réagir, à des températures comprises entre 0 et 100 °C, avec un chlorure d'alcanoyle en $C_2$-$C_4$ ou un anhydride d'alcanoyle en $C_2$-$C_4$, éventuellement en présence d'un solvant ou dilant et éventuellement d'une base organique ou inorganique et éventuellement d'un catalyseur d'acylation, ou bien on fait réagir, à des températures comprises entre 0 et 100 °C, l'alcool tertiaire, ou son sel alcalin ou son sel d'ammonium quaternaire, avec un agent d'alkylation de formule IV

$$L—R^2 \qquad (IV)$$

dans laquelle $R^2$ représente un alkyle en $C_1$-$C_4$, un alcényle en $C_2$-$C_4$ ou un alcinyle en $C_2$-$C_4$ et L un groupe éliminable pouvant subir un déplacement nucléophile, éventuellement en présence d'un solvant ou diluant et éventuellement d'une base organique ou inorganique et éventuellement d'un accélérateur de réaction, et les composés de formule I ainsi obtenus sont éventuellement transformés ensuite en leurs sels d'addition d'acide ou complexes métalliques acceptables pour les plantes.

**Revendications** (pour l'Etat contractant AT)

1. Fongicide contenant un dérivé d'azole de formule I

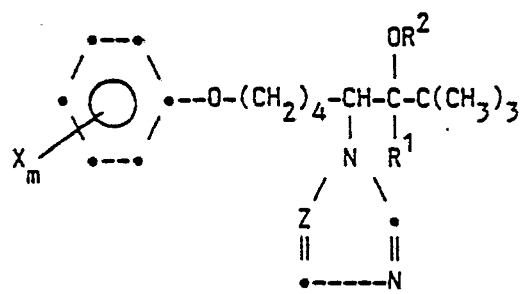

(I)

dans laquelle

X représente hydrogène, halogène ou trifluorométhyle et m un nombre entier de 1 à 5, les différents groupes X étant identiques ou différents quand m est supérieur à 1,

Z représente N ou CH,

$R^1$ représente un alcényle en $C_2$-$C_4$ ou un alcinyle en $C_2$-$C_4$ et

$R^2$ représente hydrogène, alkyle en $C_1$-$C_4$, alcényle en $C_2$-$C_4$, alcinyle en $C_2$-$C_4$ ou alcanoyle en $C_2$-$C_4$

ainsi que ses sels d'addition d'acide et complexes métalliques, compatibles avec des plantes.

2. Fongicide contenant un support solide ou liquide et un dérivé d'azole selon la revendication 1.

3. Procédé de préparation d'un fongicide, caractérisé par le fait qu'on mélange un support solide ou liquide avec un dérivé d'azole selon la revendication 1.

4. Procédé de lutte contre les champignons, caractérisé par le fait qu'on traite les champignons ou les objets à protéger contre les champignons avec un dérivé d'azole selon la revendication 1.

5. Procédé de préparation d'un dérivé d'azole selon la revendication 1 caractérisé par le fait qu'on fait réagir, éventuellement en présence d'un solvant ou diluant et éventuellement en présence d'un halogénure de magnésium ou de tétraalkylammonium, à des températures comprises entre 0 et 100 °C, une cétone de formule II

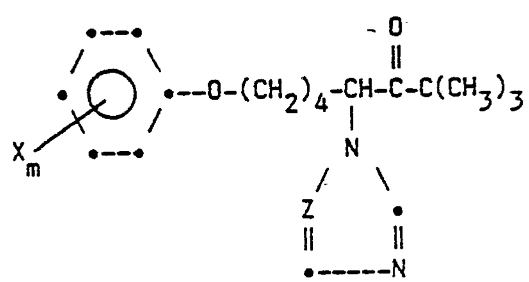

(II)

dans laquelle X, m et Z ont les significations données dans la revendication 1, avec un composé de Grignard de formule III

$$R^1\text{—MgHal} \quad \text{(III)}$$

dans laquelle $R^1$ représente alcényle en $C_2$-$C_4$ ou alcinyle en $C_3$-$C_4$ et où Hal représente chlore, brome ou iode, et l'alcoolat ainsi obtenu est ensuite hydrolysé en alcool tertiaire et l'alcool tertiaire ainsi obtenu — quand $R^2$ est différent d'hydrogène — est mis à réagir, à des températures comprises entre 0 et 100 °C, avec un chlorure d'alcanoyle en $C_2$-$C_4$ ou un anhydride d'alcanoyle en $C_2$-$C_4$, éventuellement en présence d'un solvant ou diluant et éventuellement d'une base organique ou inorganique et éventuellement d'un catalyseur d'acylation, ou bien on fait réagir, à des températures comprises entre 0 et 100 °C, l'alcool tertiaire ou son sel alcalin ou son sel d'ammonium quaternaire, avec un agent d'alkylation de formule IV

$$L\text{—}R^2 \quad \text{(IV)}$$

dans laquelle $R^2$ représente un alkyle en $C_1$-$C_4$, un alcényle en $C_2$-$C_4$ ou un alcinyle en $C_2$-$C_4$ et L un groupe éliminable pouvant subir un déplacement nucléophile, éventuellement en présence d'un solvant ou diluant et éventuellement d'une base organique ou inorganique et éventuellement d'un accélérateur de réaction, et les composés de formule I ainsi obtenus sont éventuellement transformés ensuite en leurs sels d'addition d'acide ou complexes métalliques acceptables pour les plantes.